# EUROPEAN PATENT SPECIFICATION

(11) **EP 2 897 683 B1**
(45) Date of publication and mention of the grant of the patent: **13.09.2017**
(21) Application number: 13773929.8
(22) Date of filing: 17.09.2013
(51) Int. Cl.: A61N 1/05

(54) **MEDICAL DEVICE ANCHORING SYSTEMS**
SYSTEME ZUR VERANKERUNG MEDIZINISCHER VORRICHTUNGEN
SYSTÈMES D'ANCRAGE DE DISPOSITIF MÉDICAL

(30) Priority: 19.09.2012 US 201261703044 P
(43) Date of publication of application: 29.07.2015
(73) Proprietor: Boston Scientific Neuromodulation Corporation, Valencia, CA 91355 (US)
(72) Inventor: BENTLEY, Ishmael, Eagan, MN 55122 (US); WALES, Lawrence, W., Maplewood, MN 55119 (US); PETERS, Jeffrey, J., Excelsior, Minnesota 55331 (US)
(74) Representative: Vossius & Partner Patentanwälte Rechtsanwälte mbB
(86) International application number: PCT/US2013/060158
(87) International publication number: WO 2014/047062

(56) References cited:
- EP-A1- 1 475 123
- US-A- 5 628 780
- US-A1- 2009 112 301
- US-A1- 2011 022 142
- US-A1- 2012 150 202
- US-A1- 2012 232 624
- US-A1- 2012 232 627
- US-B1- 6 554 802

## Description

### TECHNICAL FIELD

The present invention relates to medical devices for implanting implantable medical devices. More specifically, the invention relates to devices for securing implantable medical devices to a patient's anatomy.

### BACKGROUND

The implantation of various implantable medical devices requires securing the device to the patient's anatomy to prevent, or at least inhibit, unintended movement and/or migration of the implanted device. Exemplary such implantable medical devices include spinal stimulation leads, which are typically implanted adjacent to the patient's vertebral column and coupled to an implantable stimulator to provide selective nerve stimulation for pain management and the like. Such leads typically include a flexible insulative body and a compressible anchoring member disposed about the lead body. In a conventional implantation procedure, sutures or other ligatures manually tied around the anchoring member to secure the anchoring member, and consequently the lead disposed therein, to soft tissues proximate the patient's spinal column. In these applications, the anchoring member operates to delimit compressive forces on the lead itself imposed by the fixation sutures/ligatures.

The present invention is defined by the features of the claims.

U.S. Patent Application Publication No. 2012/0232627 describes an anchor including a deformable anchor sleeve with a lumen sized to receive the therapy delivery element. An outer surface of the anchor sleeve including one or more annular compression grooves oriented generally co-axial to the lumen. At least one compression member is located in a compression groove in an open configuration. One compression member for use in the pre-sutured anchor includes two portions. One portion has a plurality of protrusions that engage with corresponding recesses on the other portion.

### BRIEF DESCRIPTION OF THE DRAWINGS

FIGS. 1A-1C are perspective illustrations of an anchor and suture assembly according to one aspect of the disclosure.
FIGS. 2 and 3 are perspective illustrations of anchor examples utilizing pre-formed strain relief features according to additional aspects of the disclosure.
FIGS. 4A-4D are perspective illustrations of an anchor assembly according to an aspect of the invention.
FIGS. 5A-5J are perspective illustrations of trial anchor embodiments according to a further aspect of the invention.

### DETAILED DESCRIPTION

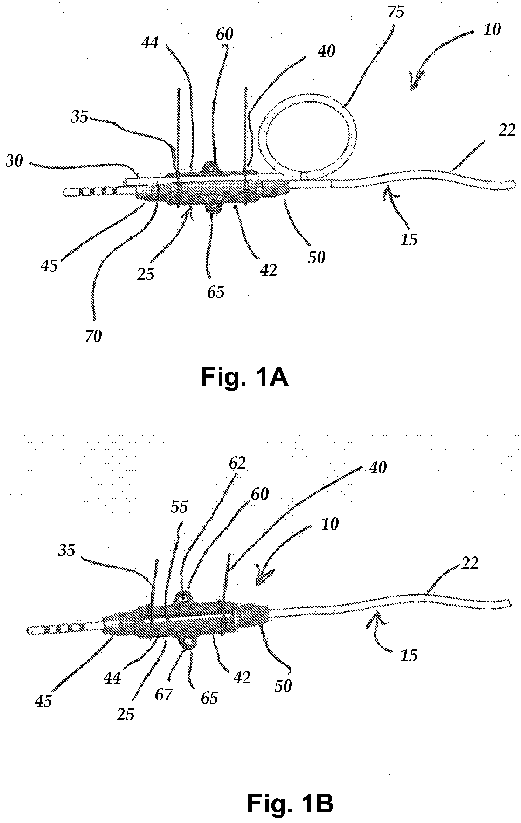

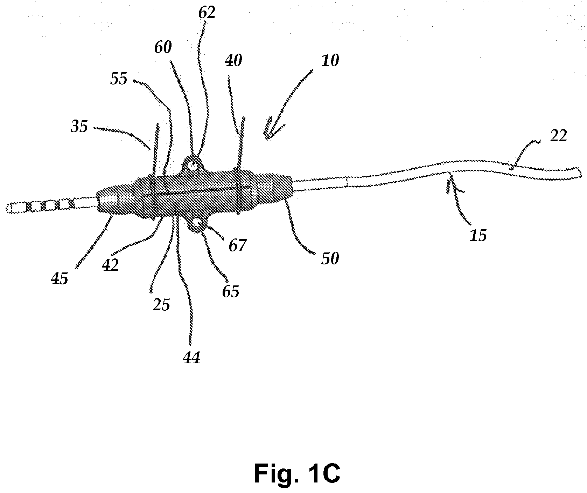

FIGS. 1A-1C are perspective illustrations of an anchor assembly 10 according to one aspect of the disclosure. The anchor assembly 10 can, in various embodiments, be used to secure an implantable device, such as an implantable electrode assembly 15, to soft tissues of a patient's body. As shown, the electrode assembly 15 has an elongate flexible body 22. As further shown, the anchor assembly 10 includes an anchoring member 25, a removable pin 30, and a pair of pre-tied suture loops 35, 40 disposed about the anchoring member 25.

In the illustrated embodiment, the anchoring member 25 has a body 42 with an outer surface 44 and a longitudinal lumen (not shown) through which a portion of the body 22 of the electrode assembly 15 is disposed. In addition, the anchoring member 25 includes a first end 45, an opposite second end 50, a longitudinal slot 55, a wing member 60 including a through hole 62, and a wing member 65 including a through hole 67. As shown, the wing members 60, 65 extend radially from the body 42 and are disposed generally diametrically opposite one another. In the various embodiments, the wing members 60, 65 operate to cooperate with a portion of a tissue fixation element, e.g., a suture loop or a suture/tissue anchor assembly, to secure the anchoring member 25. In such embodiments, the suture and/or tissue anchor can be inserted through the through hole 62 and/or 67 in the respective wing member 60, 65 and into the tissue to which the anchoring member 25 is desired to be affixed.

As further shown, the longitudinal slot 55 extends from proximate the first end 45 to proximate the second end 50, and further extends radially inward from the outer surface 44 to the longitudinal lumen, i.e., through the outer wall of material making up the body 42 of the anchoring member 25. Additionally, in the illustrated embodiments, the suture loops 35, 40 are disposed about the anchoring member 25 proximate the first end 45 and the second end 50 of the anchoring member body 42. In various embodiments, the suture loops 35, 40 can be positioned within circumferential grooves (not shown in FIGS. 1A-1C) in the body 42 of the anchoring member 25 to enhance the engagement and alignment of the suture loops 35, 40. In other embodiments, however, no such circumferential grooves are provided in the anchoring member body 42.

As shown in a comparison of FIGS. 1A and 1B, the pin 30 has a longitudinal segment 70 disposed within the slot 55 of the anchoring member 25, and is removable therefrom, e.g., by grasping and manipulating a handle portion 75 to separate the pin 30 from the anchoring member 25. In various embodiments, the pin 30 operates to maintain the anchoring member 25, and in particular, the body 42 thereof, in an open configuration in which the lumen is sized to slidably receive a portion of the body 22 of the electrode assembly 15. Subsequently, upon removal of the pin 30 the body 42 of the anchoring member 25 is radially compressible so as to apply a radially inward-directed force on the portion of the body 22 of the electrode assembly 15 disposed within the anchoring member lumen, thereby inhibiting movement of the electrode assembly body 22 relative to the anchoring member 25. In various embodiments, the suture loops 35, 40 can be tightened to radially compress the anchoring member 25 as described above. The anchoring member 25 thus operates in part to distribute the radial forces imposed by the suture loops 35, 40, to avoid excessive stress on the body 22 of the electrode assembly 15. In various embodiments, the slot 55 operates as a relief feature, which allows the body 22 of the electrode assembly to freely move through the anchoring member body 42 when the suture loops 35, 40 are not secured. Once the electrode assembly body 22 is situated as desired by the clinician, the pin 30 can be removed and the suture loops 35, 40 tightened to compress the anchoring member body 42 against the electrode assembly body 22, securing the electrode assembly 15 with respect to the anchoring member 25. Thereafter, as described above, a fixation element (e.g., a suture loop or suture/anchor assembly) can be placed across the anchoring member body 42 and through the through holes 62, 67 to secure the anchoring member to the tissue.

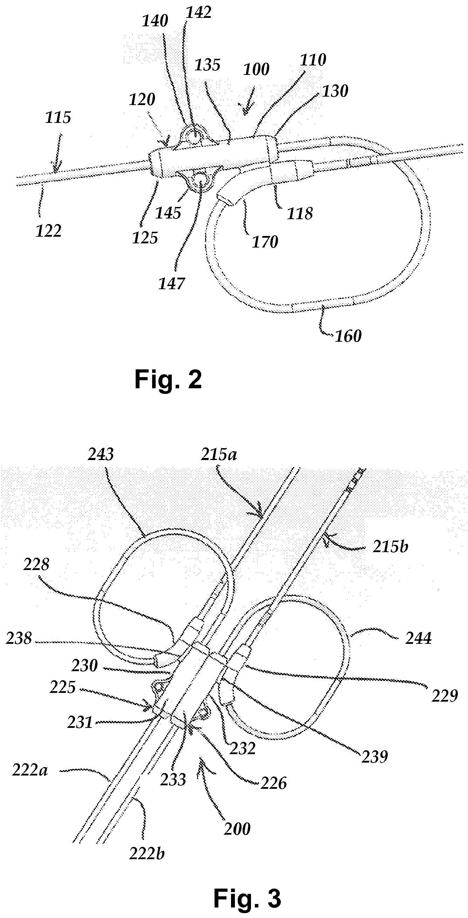

FIG. 2 is a perspective illustration of an anchor assembly 100 utilizing a pre-formed strain relief feature according to another aspect of the disclosure. As shown in FIG. 2, the anchor assembly 100 includes an anchoring member 110 and a strain relief member 118. As further shown, the anchoring member 110 has a body portion 120 with opposite ends 125, 130 and an outer surface 135. Additionally, the anchoring member 110 includes a wing member 140 including a through hole 142, and a wing member 145 including a through hole 147. As shown, the wing members 140, 145 extend radially from the body portion 120 and are disposed generally diametrically opposite one another. As with the anchoring member 25 discussed above, in the various embodiments, the wing members 140, 145 operate to cooperate with a portion of a tissue fixation element, e.g., a suture loop or a suture/tissue anchor assembly, to secure the anchoring member 110 to the patient's tissue. The anchoring member 110 further includes a longitudinal lumen (not shown) extending through the ends 125, 130, the lumen being sized to slidably receive a portion of a body 122 of an implantable electrode assembly 115 therethrough. As with the anchoring member 25 described above, the anchoring member 110 is configured to be radially compressible to inhibit relative sliding motion with respect to the portion of the body 122 of the electrode assembly 115 disposed within the lumen of the anchoring member.

As further shown, the strain relief member 118 is attached to and positioned adjacent to the anchoring member 110. The strain relief member 118 also includes a longitudinal lumen (not shown) sized to slidably receive a portion of the body 122 of the implantable electrode assembly 115. As further shown, the strain relief member 118 is oriented to facilitate formation of a strain relief loop 160 in the body 122 of the electrode assembly 115 between the portions disposed, respectively, within the lumen of the anchoring member 110 and the strain relief member 118. As further shown, the strain relief member 118 includes a pre-curved portion 170 disposed adjacent to the body portion 120 of the anchoring member 110 between the ends 125, 130 thereof. The pre-curved portion 170 further facilitates forming the strain relief loop 160 in the body 122 of the electrode assembly 115.

In use, the anchoring member 110 is configured to operate as an anchoring element that forms a friction fit against the body 122 of the electrode assembly 115 disposed within the lumen of the anchoring member 110. The body portion 120 of the anchoring member 110 is configured to be sutured and secured to tissue (e.g., muscle, fascia, connective tissue, or bony) to secure the electrode assembly 115 in the desired location. At the same time, the strain relief member 118 is configured to facilitate formation of and maintenance of the strain relief loop 160 in the electrode assembly body 122. In the various embodiments, the lumen of the strain relief element 118 is configured to provide a slip fit against the body 122 of the electrode assembly 115, which allows the strain relief loop 160 to move, isolating the proximal end of the electrode assembly 115 from its distal end, which is positioned by the clinician so the electrodes will deliver current to the proper location.

FIG. 3 is a perspective illustration of an anchor assembly 200 utilizing a pre-formed strain relief feature according to another aspect of the disclosure. As shown, the anchor assembly 200 includes a pair of anchoring members 225, 226 and a pair of strain relief members 228, 229. As further shown, the anchoring member 225 has a body portion 230 with an outer surface 231, and the anchoring member 226 has a body portion 232 with an outer surface 233. Additionally, the strain relief member 228 is attached to the anchoring member 225 via a connecting segment 238, and the strain relief member 229 is attached to the anchoring member 226 via a connecting segment 239. As can be seen in FIG. 3, the anchoring member body portions 230, 232 are attached to one another so as to form a unitary structure.

In the various embodiments, the anchoring members 225, 226 and corresponding strain relief members 228, 229 are configured in substantially the same way as the anchoring member 110 and the strain relief member 118 of the anchor assembly 100 described above, and thus have substantially the same or identical functionality. That is, the anchoring member 225 is configured to facilitate securing an electrode assembly 215a to tissue, and the anchoring member 226 is configured to facilitate securing an electrode assembly 215b to tissue. In addition, the anchoring member 225 is configured to receive and frictionally engage a portion of a body 222a of the electrode assembly 215a, while the strain relief member 228 is configured to slidably receive and facilitate formation of a strain relief loop 243 in the body 222a of the electrode assembly 215a. Similarly, the anchoring member 226 is configured to receive and frictionally engage a portion of a body 222b of the electrode assembly 215b, while the strain relief member 229 is configured to slidably receive and facilitate formation of a strain relief loop 244 in the body 222b of the electrode assembly 215b. The anchoring assembly 200 thus provides an efficient, compact means for anchoring a pair of electrode assemblies to tissue while facilitating the formation of a strain relief loop in each of the electrode assemblies to enhance the reliability and long term stability of the implanted electrode assemblies. In various embodiments, the anchoring assembly 200 can be manufactured in a unitary form - e.g., from a single molding. In other embodiments, each of the respective anchoring members and strain relief members can be separately formed and subsequently assembled and attached together to form the complete anchoring assembly 200.

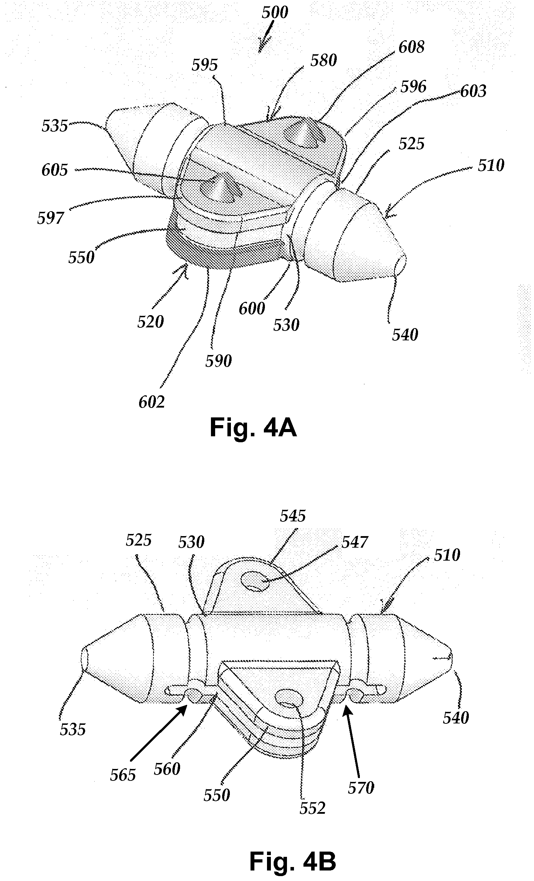

FIGS. 4A-4D are perspective illustrations of an anchor assembly 500 according to an aspect of the invention. As shown in FIGS. 4A-4D, the anchor assembly 500 includes an anchoring member 510 and an outer shell assembly 520. As explained in further detail below, in the various embodiments, the anchoring member 510 is resilient and compressible, and is configured to receive a portion of a medical device, e.g., the electrode assembly 15 illustrated in FIGS. 1A-1C, and to facilitate securing the device to a patient's tissues. Additionally, the shell assembly 520 is operable to radially compress the anchoring member 510 about the medical device disposed therein to inhibit relative movement of the medical device and the anchoring member, such as described above with respect to the various other embodiments of anchoring members and anchor assemblies. In various embodiments, the anchoring member 510 can be used as a standalone anchoring system - i.e., without the use of the shell assembly 520.

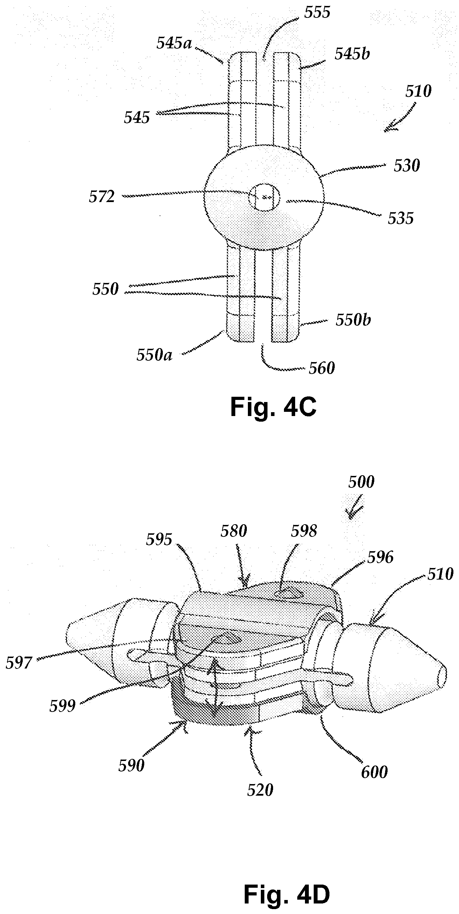

FIGS. 4B and 4C, respectively, are isometric and end views of the anchoring member 510. As shown in FIGS. 4B and 4C, the anchoring member 510 has a body portion 525 with an outer surface 530 and includes opposite open ends 535, 540. The anchoring member 510 further includes a wing member 545 with a through hole 547, and a wing member 550 with a through hole 552, a longitudinal slot 555 and a longitudinal slot 560. As further shown, in the illustrated embodiment, the body portion 525 includes a pair of circumferential grooves 565, 570 disposed between the ends 535, 540. The anchoring member further includes a longitudinal lumen 572 through which a portion of the medical device (e.g., the body 22 of the electrode assembly 15) can be received. In various embodiments, the circumferential grooves 565, 570 operate as locations for suture loops or suture/tissue anchor assemblies to engage the anchoring member 510 for securing the anchoring member 510 to the patient's tissue. Additionally, the same suture loops and/or suture/anchor assemblies may assist in radially compressing the medical device disposed within the lumen 572. In various other embodiments, however, the circumferential grooves 565, 570 are omitted.

As shown, the wing members 545, 550 and the longitudinal slots 555, 560 are disposed substantially diametrically opposite one another. As further shown, the longitudinal slots 555, 560 each extend from proximate the end 535 to proximate the end 540 radially through the wing members 545, 550, respectively and the outer surface 530 into the lumen 572. As can be seen in FIG. 4C, the slots 555, 560 bisect the wing members 545, 550, respectively, to form wing member portions 545a, 545b and 550a, 550b, respectively. The slots 555, 560 operate as relief features to allow for slidable movement of the body 22 of the electrode assembly 15 (FIGS. 1A-1C) within the lumen 572 prior to radial compression of the body portion 525, and also to aid in the compressibility of the body portion 525 to frictionally engage the body 22 of the electrode assembly.

As further shown, particularly in FIGS. 4A and 4D, the shell assembly 520 includes mating shell portions 580, 590 which can be disposed on opposite sides of the anchoring member 510 and which can be coupled together and cooperate to radially compress the anchoring member 510 so inhibit relative movement of the anchoring member 510 and the portion of the electrode assembly disposed therein. As shown, in the illustrated embodiment, the shell portion 580 includes a center portion 595 and lateral portions 596, 597 each having, respectively, an aperture 598, 599 therethrough. Additionally, the shell portion 590 includes a center portion 600 and lateral portions 602, 603 each including, respectively, a prong member 605, 608 extending therefrom. As can be seen in FIGS. 4A and 4D, the center portions 595, 600 have generally semi-cylindrical shapes that are complimentary to the generally cylindrical shape of the body 525 of the anchoring member 510. Additionally, the lateral portions 596, 597, 602 and 603 have shapes that generally match and complement the shapes of the wing members 545, 550 of the anchoring member 510.

In the assembled anchor assembly 500, the shell portions 580, 590 are positioned opposite one another with the anchoring member 510 sandwiched therebetween. Additionally, the prong members 605, 608 extend through the through holes 552, 547, respectively of the wing members 550, 545 of the anchoring member 510, and further through the apertures 599, 598 of the shell portion 580. The assembled shell portions 580, 590 operate to radially compress the body portion 525 of the anchoring member 510 about a portion of an implantable device disposed within the lumen 572 of the anchoring member 510. In the various embodiments, each of the prong members 605, 608 includes a resilient end portion that can radially expand to a diameter larger 599, 598 through which it is fully inserted, so as to inhibit spontaneous separation of the portions 580, 590 of the outer shell assembly 520 once engaged. The prong members 605, 608 and the corresponding apertures 599, 598 thus form a locking arrangement for maintain the shell assembly 520 in its assembled state, which locking arrangement is reversible (e.g., by an appropriately designed instrument) to allow the shell assembly 520 to be separated, e.g., to reposition the anchoring member 510 as desired.

The various anchoring members described herein can be made from a variety of resilient, biocompatible materials, e.g., silicone rubber, polyurethane, polyether block amides, and the like. In various embodiments, when present, the shell assembly 520 of the anchoring assembly 500 can be made of a biocompatible, relatively rigid or semi-rigid polymeric or metallic materials such as, for example, polyether etherketone (PEEK™), polyethylene teraphthalate (PET), acrylics, polycarbonates, engineering plastics; and/or composites, as well as nickel, titanium, and alloys thereof.

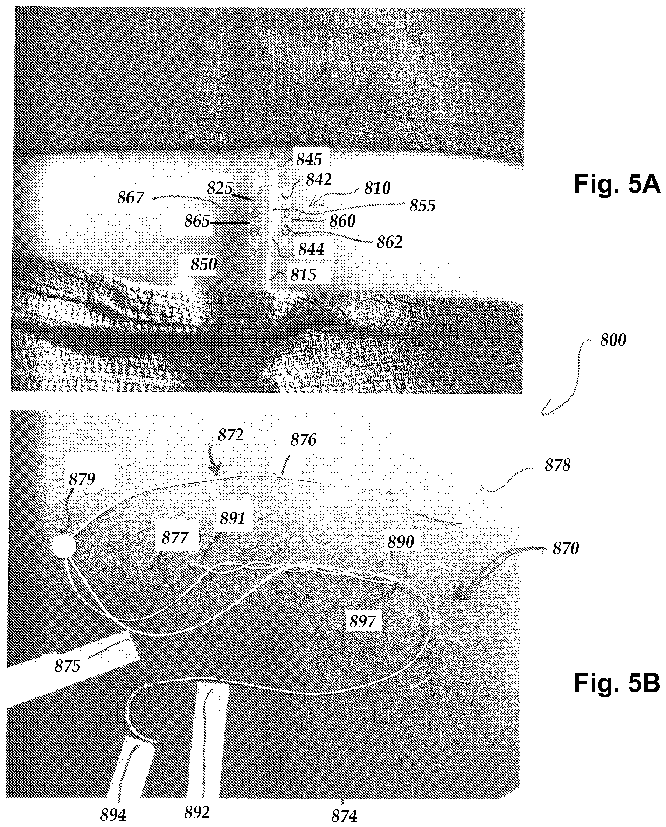

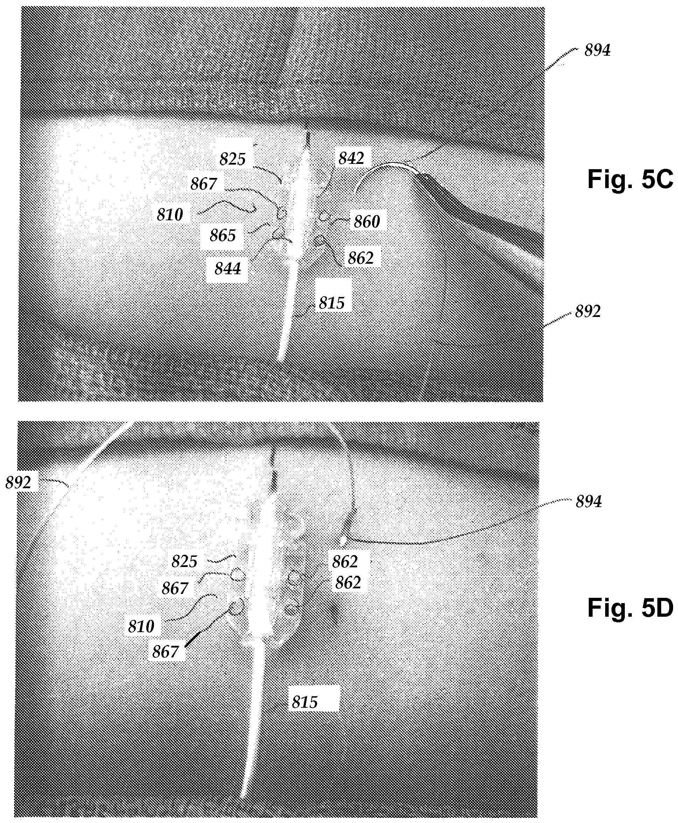

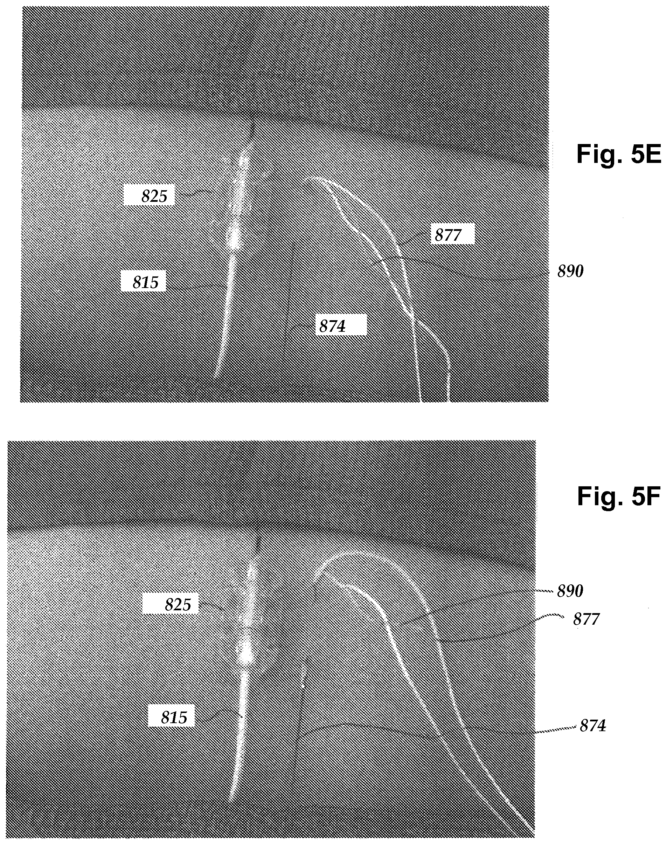

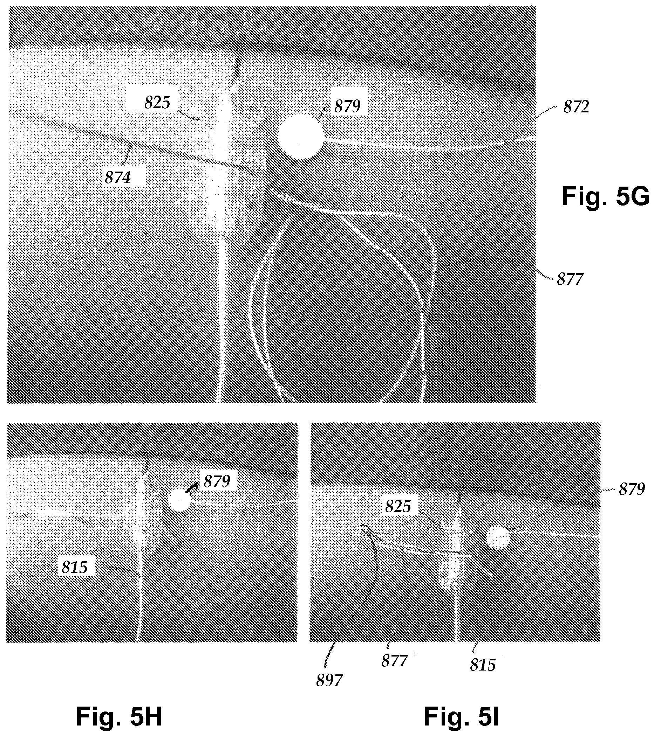

Referring now to FIGS. 5A-5J a perspective illustration of a trail anchor and suture system 800 in accordance to a further aspect of the invention is shown. Trial anchors 810 are used primarily prior to a surgical procedure to implanting the anchor in situations in which it is desirable to test the therapy on a patient on a short term basis. The anchoring member 825 is substantially similar to other anchor members disclosed herein and includes a body 842 with an outer surface 844 and a longitudinal lumen (not shown) through which a portion of the body 822 of the electrode assembly 815 is disposed. In addition, the anchoring member 825 includes a first end 845, an opposite second end 850, a longitudinal slot (not shown), and one or more wing members 860, 865 including one or more through holes 862, 867. As shown in FIG. 5A, the wing members 860, 865 extend radially from the body 842 and are disposed generally diametrically opposite one another. In the various embodiments, the wing members 860, 865 operate to cooperate with a portion of a suture assembly 870, to secure the anchoring member 825. As will hereinafter be described, the suture and/or tissue anchor can be inserted through the through holes 862 and/or 867 in the respective wing member 860, 865 and into the tissue to which the anchoring member 825 is temporarily desired to be affixed.

Suture assembly 870 broadly includes a first suture portion 872 and tissue anchor portion 874. First suture portion 872 is a continuous length of suture forming a distal suture element 875 and proximal suture element 876. Proximal suture element 876 terminates in knotted loop 878 at the proximal end thereof which functions as a manual pull. As can be best seen in FIG. 5B distal suture element 875 forms a continuous loop 877 of suture material. An adjustable locking knot 897, such as a Weston or Roeder knot or other such adjustable knots known to those of skill in the art, completes the continuous suture loop 877. Sliding the knot will shorten the suture loop 877. The disc 879 has a through hole in which the suture loop is allowed to freely slide through. Terminating end (not shown) of continuous loop is operably secured by disc 879 while proximal sutured portion 876 may freely move within disc 879. The proximal end 890 of tissue anchor portion 874 is woven through continuous loop 877 in a serpentine fashion and includes tail end 891. Distal end 892 of tissue anchor portion 874 terminates in suture needle 894.

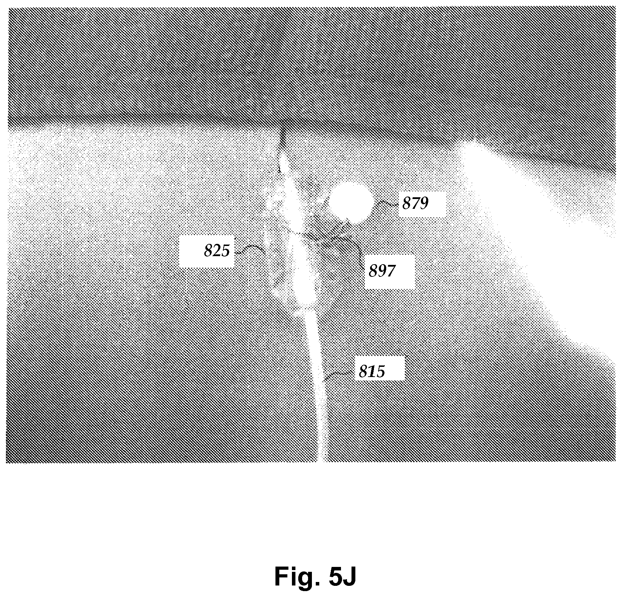

Referring now to FIGS. 5C - 5J, in operation the surgeon inserts suture needle 894 through patient's skin adjacent trial anchor 810 and pulls suture 892 through skin until disc 879 lies adjacent patient's skin thereby preventing further suture being threaded through patient's skin as best seen in FIG. 5G. The clinician then inserts suture portion 874 and distal suture element 875 until continuous loop 877 and tail end 891 are pulled almost but not completely past through hole 862 and/or 867 and the tail end 891. The clinician then manually pulls on proximal suture element 876 of first suture portion 872 via knotted loop 878 which in turn causes continuous loop portion 877 to retract and pull on serpentine tissue anchor portion 874. As the retraction of continuous loop portion 877 continues, serpentine proximal end 890 of tissue anchor 874 forms knot 896 which secures trial anchor 810 in place on patient's skin as best seen in FIG. 5J. The clinician then cuts or trims first suture portion 872 and close to the patient's skin and trims tissue anchor portion close to knot 896. Those of skill in the art will appreciate that the process may be repeated to secure additional through holes 862, 867 as desired.

Although the present invention has been described with reference to various embodiments, those of ordinary skill in the art will recognize that changes may be made in form and detail without departing from the scope of the inventions disclosed herein which is defined by the appended claims.

## Claims

1. An anchor assembly, comprising:
an anchoring member (510) comprising a body portion (525) having a first end (535) and a second end (540) opposite the first end, the anchoring member defining a longitudinal lumen (572) in the body portion extending from the first end to the second end and configured and arranged to slidably receive a portion of an electrode assembly (15), wherein the anchoring member further comprises two opposing wing members (545, 550) extending from the body portion, each of the wing members defining a through hole (547, 552); and
a shell assembly (520) comprising first and second mating shell portions (590, 580) configured and arranged to be disposed on opposites sides of the anchoring member and to be coupled together to anchor the portion of the electrode assembly within the anchoring member, each of the first and second mating shell portions comprising a center portion (600, 595) and two lateral portions (602, 603, 596, 597) extending from the center portion, wherein the center portion fits over the anchoring member with the lateral portions of the first and second mating shell portions opposite each other, wherein the lateral portions of the first mating shell portion each have a prong member (605, 608) and the lateral portions of the second mating shell portion each have an aperture (598, 599), wherein the prong members and apertures are configured and arranged for mating to form a locking arrangement, wherein the shell assembly (520) is configured and arranged so that the prong members (605, 508) of the lateral portions of the first mating portion pass through the through holes (547, 552) of the two opposing wing members (545, 550) when the shell assembly (520) is disposed on opposite sides of the anchoring member (510) and the first and second mating portions are mated.

2. The anchor assembly of claim 1, wherein the body portion of the anchoring member defines a longitudinal slot (555) through an outer surface of the body portion and extending radially inward to the longitudinal lumen.

3. The anchor assembly of claim 1, wherein each wing member comprises two opposing wing member portions (545a, 545b, 550a, 550b) separated by a bisecting slot (555, 560).

4. The anchor assembly of claim 3, wherein the bisecting slot of at least one wing member extends to the longitudinal slot of the body portion of the anchoring member.

5. The anchor assembly of claim 3, wherein the lateral portions of the first and second mating portions of the shell assembly are configured and arranged to be positioned over the wing member portions of the anchoring member and to radially compress the body portion and the wing member portions of the anchoring member when the first and second mating portions are mated.

6. The anchor assembly of claim 1, wherein the body portion of the anchoring member defines two longitudinal slots (555, 560) through an outer surface of the body portion and extending radially inward to the longitudinal lumen, wherein the two longitudinal slots are defined diametrically opposite each either on the body portion.

7. The anchor assembly of claim 1, wherein the lateral portions of the first and second mating portions of the shell assembly are configured and arranged to be positioned over the wing members of the anchoring member.

8. The anchor assembly of claim 1, wherein the body portion comprises a pair of circumferential grooves (565, 570) disposed between the first and second ends.

9. The anchor assembly of claim 1, wherein the body portion of the anchoring member has a cylindrical shape and the center portions of the first and second mating shell portions each have a semi-cylindrical shape complimentary to the cylindrical shape of the body portion.

10. An anchoring system, comprising
the anchor assembly (500) of any one of claims 1-9 and
an electrode assembly (15) comprising an elongate flexible body (22), wherein the anchoring member is configured and arranged to receive a portion of the electrode assembly.

## Patentansprüche

1. Ankeranordnung, die aufweist:
ein Verankerungsteil (510), das einen Körperabschnitt (525) mit einem ersten Ende (535) und einem zweiten Ende (540) entgegengesetzt zum ersten Ende aufweist, wobei das Verankerungsteil ein Längslumen (572) im Körperabschnitt definiert, das sich vom ersten Ende zum zweiten Ende erstreckt und so konfiguriert und angeordnet ist, dass es einen Abschnitt einer Elektrodenanordnung (15) gleitfähig aufnimmt, wobei das Verankerungsteil ferner zwei gegenüberliegende Flügelteile (545, 550) aufweist, die sich vom Körperabschnitt erstrecken, wobei jedes der Flügelteile ein Durchgangsloch (547, 552) definiert; und
eine Schalenanordnung (520) mit einem ersten und einem zweiten Gegenschalenabschnitt (590, 580), die so konfiguriert und angeordnet sind, dass sie auf Gegenseiten des Verankerungsteils liegen und miteinander gekoppelt sind, um den Abschnitt der Elektrodenanordnung im Verankerungsteil zu verankern, wobei der erste und zweite Gegenschalenabschnitt jeweils einen Mittelabschnitt (600, 595) und zwei sich vom Mittelabschnitt erstreckende Seitenabschnitte (602, 603, 596, 597) aufweisen, wobei sich der Mittelabschnitt über dem Verankerungsteil so aufpasst, dass die Seitenabschnitte des ersten und zweiten Gegenschalenabschnitts entgegengesetzt zueinander sind, wobei die Seitenabschnitte des ersten Gegenschalenabschnitts jeweils ein Stiftteil (605, 608) haben und die Seitenabschnitte des zweiten Gegenschalenabschnitts jeweils eine Öffnung (598, 599) haben, wobei die Stiftteile und Öffnungen zum Zusammenpassen so konfiguriert und angeordnet sind, dass sie eine Verriegelungsanordnung bilden, wobei die Schalenanordnung (520) so konfiguriert und angeordnet ist, dass die Stiftteile (605, 608) der Seitenabschnitte des ersten Gegenabschnitts die Durchgangslöcher (547, 552) der beiden gegenüberliegenden Flügelteile (545, 550) durchlaufen, wenn die Schalenanordnung (520) auf Gegenseiten des Verankerungsteils (510) angeordnet ist und der erste und zweite Gegenabschnitt zusammengepasst sind.

2. Ankeranordnung nach Anspruch 1, wobei der Körperabschnitt des Verankerungsteils einen Längsschlitz (555) durch eine Außenfläche des Körperabschnitts und sich radial nach innen zum Längslumen erstreckend definiert.

3. Ankeranordnung nach Anspruch 1, wobei jedes Flügelteil zwei gegenüberliegende Flügelteilabschnitte (545a, 545b, 550a, 550b) aufweist, die durch einen Halbierungsschlitz (555, 560) getrennt sind.

4. Ankeranordnung nach Anspruch 3, wobei sich der Halbierungsschlitz mindestens eines Flügelteils zum Längsschlitz des Körperabschnitts des Verankerungsteils erstreckt.

5. Ankeranordnung nach Anspruch 3, wobei die Seitenabschnitte des ersten und zweiten Gegenabschnitts der Schalenanordnung so konfiguriert und angeordnet sind, dass sie über den Flügelteilabschnitten des Verankerungsteils positioniert sind und den Körperabschnitt sowie die Flügelteilabschnitte des Verankerungsteils radial zusammendrücken, wenn der erste und zweite Gegenabschnitt zusammengepasst sind.

6. Ankeranordnung nach Anspruch 1, wobei der Körperabschnitt des Verankerungsteils zwei Längsschlitze (555, 560) durch eine Außenfläche des Körperabschnitts und sich radial nach innen zum Längslumen erstreckend definiert, wobei die beiden Längsschlitze auf dem Körperabschnitt diametral entgegengesetzt zueinander definiert sind.

7. Ankeranordnung nach Anspruch 1, wobei die Seitenabschnitte des ersten und zweiten Gegenabschnitts der Schalenanordnung so konfiguriert und angeordnet sind, dass sie über den Flügelteilen des Verankerungsteils positioniert sind.

8. Ankeranordnung nach Anspruch 1, wobei der Körperabschnitt ein Paar Umfangsnuten (565, 570) aufweist, die zwischen dem ersten und zweiten Ende angeordnet sind.

9. Ankeranordnung nach Anspruch 1, wobei der Körperabschnitt des Verankerungsteils eine Zylinderform hat und die Mittelabschnitte des ersten und zweiten Gegenschalenabschnitts jeweils eine Halbzylinderform komplementär zur Zylinderform des Körperabschnitts haben.

10. Verankerungssystem, das aufweist:
die Ankeranordnung (500) nach einem der Ansprüche 1 bis 9 und
eine Elektrodenanordnung (15) mit einem länglichen flexiblen Körper (22), wobei das Verankerungsteil so konfiguriert und angeordnet ist, dass es einen Abschnitt der Elektrodenanordnung aufnimmt.

## Revendications

1. Ensemble d'ancrage comprenant :
un élément d'ancrage (510) comprenant une partie de corps (525) ayant une première extrémité (535) et une seconde extrémité (540) opposée à la première extrémité, l'élément d'ancrage définissant une lumière longitudinale (572) dans la partie de corps s'étendant de la première extrémité à la seconde extrémité et configurée et agencée pour recevoir, de manière coulissante, une partie d'un ensemble d'électrode (15), dans lequel l'élément d'ancrage comprend en outre deux éléments d'aile (545, 550) opposés s'étendant à partir de la partie de corps, chacun des éléments d'aile définissant un trou débouchant (547, 552) ; et
un ensemble de coques (520) comprenant des première et seconde parties de coque de couplage (590, 580) configurées et agencées pour être disposées sur des côtés opposés de l'élément d'ancrage et être couplées ensemble pour ancrer la partie de l'ensemble d'électrode à l'intérieur de l'élément d'ancrage, chacune des première et seconde parties de coque de couplage comprenant une partie centrale (600, 595) et deux parties latérales (602, 603, 596, 597) s'étendant à partir de la partie centrale, dans lequel la partie centrale se monte sur l'élément d'ancrage avec les parties latérales des première et seconde parties de coque de couplage opposées entre elles, dans lequel les parties latérales de la première partie de coque de couplage ont chacune un élément de dent (605, 608) et les parties latérales de la seconde partie de coque de couplage ont chacune une ouverture (598, 599), dans lequel les éléments de dent et les ouvertures sont configurés et agencés pour se coupler afin de former un agencement de verrouillage, dans lequel l'ensemble de coques (520) est configuré et agencé de sorte que les éléments de dent (605, 608) des parties latérales de la première partie de couplage passent à travers les trous débouchants (547, 552) des deux éléments d'aile (545, 550) opposés lorsque l'ensemble de coques (520) est disposé sur les côtés opposés de l'élément d'ancrage (510) et que les première et seconde parties de couplage sont couplées.

2. Ensemble d'ancrage selon la revendication 1, dans lequel la partie de corps de l'élément d'ancrage définit une fente longitudinale (555) à travers une surface externe de la partie de corps et s'étendant radialement vers l'intérieur jusqu'à la lumière longitudinale.

3. Ensemble d'ancrage selon la revendication 1, dans lequel chaque élément d'aile comprend deux parties d'élément d'aile (545a, 545b, 550a, 550b) opposées séparées par une fente coupée en deux (555, 560).

4. Ensemble d'ancrage selon la revendication 3, dans lequel la fente coupée en deux d'au moins un élément d'aile s'étend vers la fente longitudinale de la partie de corps de l'élément d'ancrage.

5. Ensemble d'ancrage selon la revendication 3, dans lequel les parties latérales des première et seconde parties de couplage de l'ensemble de coques sont configurées et agencées pour être positionnées sur les parties d'élément d'aile de l'élément d'ancrage et pour comprimer radialement la partie de corps et les parties d'élément d'aile de l'élément d'ancrage lorsque les première et seconde parties de couplage sont couplées.

6. Ensemble d'ancrage selon la revendication 1, dans lequel la partie de corps de l'élément d'ancrage définit deux fentes longitudinales (555, 560) à travers une surface externe de la partie de corps et s'étendant radialement vers l'intérieur vers la lumière longitudinale, dans lequel les deux fentes longitudinales sont définies de manière diamétralement opposée l'une par rapport à l'autre sur la partie de corps.

7. Ensemble d'ancrage selon la revendication 1, dans lequel les parties latérales des première et seconde parties de couplage de l'ensemble de coques sont configurées et agencées pour être positionnées sur les éléments d'aile de l'élément d'ancrage.

8. Ensemble d'ancrage selon la revendication 1, dans lequel la partie de corps comprend une paire de rainures circonférentielles (565, 570) disposées entre les première et seconde extrémités.

9. Ensemble d'ancrage selon la revendication 1, dans lequel la partie de corps de l'élément d'ancrage a une forme cylindrique et les parties centrales des première et seconde parties de coque de couplage ont chacune une forme semi-cylindrique complémentaire de la forme cylindrique de la partie de corps.

10. Système d'ancrage comprenant :
l'ensemble d'ancrage (500) selon l'une quelconque des revendications 1 à 9, et
un ensemble d'électrode (15) comprenant un corps flexible allongé (22), dans lequel l'élément d'ancrage est configuré et agencé pour recevoir une partie de l'ensemble d'électrode.
